# EUROPEAN PATENT SPECIFICATION

(11) **EP 2 830 618 B1**
(45) Date of publication and mention of the grant of the patent: **27.07.2016**
(21) Application number: 13769567.2
(22) Date of filing: 22.03.2013
(51) Int. Cl.: A61K 31/4178, A61K 31/505, A61K 9/20, A61K 9/48

(54) **PHARMACEUTICAL COMPOSITION COMPRISING OLMESARTAN MEDOXOMIL AND ROSUVASTATIN OR ITS SALT**
PHARMAZEUTISCHE ZUSAMMENSETZUNG MIT OLMESARTAN-MEDOXOMIL UND ROSUVASTATIN ODER DESSEN SALZ
COMPOSITION PHARMACEUTIQUE COMPRENANT DE L'OLMÉSARTAN MÉDOXOMIL ET DE LA ROSUVASTATINE OU SON SEL

(30) Priority: 30.03.2012 KR 20120032903
(43) Date of publication of application: 04.02.2015
(73) Proprietor: Daewoong Pharmaceutical Co., Ltd, Seongnam Si, Gyeonggi-Do 462-120 (KR)
(72) Inventor: CHANG, Hee-Chul, Seoul 135-280 (KR); KANG, Bok-Ki, Yongin-si Gyeonggi-do 446-736 (KR); KIM, Jun-Ku, Yongin-si Gyeonggi-do 449-814 (KR)
(74) Representative: Turner, Craig Robert
(86) International application number: PCT/KR2013/002378
(87) International publication number: WO 2013/147462

(56) References cited:
- WO-A1-2009/057569
- WO-A2-2008/068217
- WO-A2-2009/073683
- WO-A2-2009/154810
- WO-A2-2011/107750
- US-A1- 2011 212 175
- US-A1- 2011 217 374
- US-B1- 8 399 011

## Description

### Technical Field

The present invention relates to a pharmaceutical composition having a single dosage form comprising olmesartan medoxomil and rosuvastatin or its salt.

### Background Art

There are many clinical cases having both hypertension and hyperlipidemia, which are regarded as a major risk factor to progress heart diseases and ultimately cause adverse cardiac symptoms. These clinical cases are originated from potentially common mechanism. Therefore, it is advantageous for the patients to receive a single dosage form for treating both of the diseases. For example, as a combination formulation for the treatment of both hypertension and hyperlipidemia, Caduet™, a combination formulation of atorvastatin and amlodipine, is being clinically used. In addition, various literatures disclose combinations of an antihypertensive agent and an antihyperlipidemic agent (International Publication Nos. WO95/26188, WO97/37688, WO99/11260, WO00/45818, WO04/062729, WO06/040085, etc.).

Meanwhile, rosuvastatin or its salt (for example, the calcium salt), an HMG-CoA reductase inhibitor, is being used for the treatment of hypercholesterolemia, hyper-lipoproteinemia, and atherosclerosis; and is commercially available under the trade name of Cresto™ tablet. Olmesartan medoxomil is used for the treatment of essential hypertension; and is commercially available under the trade name of Olmetec™ tablet. Unlike other statin drugs, rosuvastatin is metabolized mainly by CYP 2C9 and CYP 2C19, but only 10% thereof is metabolized. Therefore, the therapeutic effect is originated mainly from rosuvastatin *per se.* Olmesartan medoxomil is rapidly metabolized after the *in vivo* absorption thereof; but not metabolized by CYP450. Therefore, after the two drugs are absorbed into the body, it is expected that the metabolic interaction of the two drugs does not occur.

### Disclosure of Invention

### Technical Problem

The present inventors designed various formulations in order to develop a combination formulation in a single dosage form containing rosuvastatin and olmesartan medoxomil as active ingredients. Surprisingly, the present inventors found that, when rosuvastatin and olmesartan medoxomil are administered in the form of a single matrix formulation, there is drug-drug interaction and/or drug-drug interference between rosuvastatin and olmesartan medoxomil (one of the lipophilic drugs), which results in delaying the *in vivo* release (i.e., dissolution) of rosuvastatin calcium to the gastrointestinal fluid and thus delaying the translocation thereof to the gastrointestinal membrane, thereby inhibiting the absorption of rosuvastatin. That is, when a single matrix formulation containing the two drugs was administered, the projected 90% confidence interval of AUC and Cmax of rosuvastatin exceeded the bioequivalence criteria, i.e., the range of 0.8 to 1.25.

And also, the present inventors found that, even when olmesartan medoxomil and rosuvastatin or its salt were formulated into a combination dosage form having separate compartments according to a conventional formulation method, it was difficult to obtain a combination formulation bioequivalent to the single formulation of each of drugs. The present inventors found that, when the compartment comprising rosuvastatin or its salt includes a certain disintegrant, i.e., cellulose-type and/or povidone-type disintegrants, in a certain amount, rapid disintegration and high initial dissolution rate of rosuvastatin or its salt can be accomplished, thereby being able to obtain a combination formulation bioequivalent to the single formulation of rosuvastatin or its salt. Especially, the present inventors surprisingly found that, in case of olmesartan medoxomil, a combination formulation comprising rosuvastatin and olmesartan medoxomil should be designed so as to exhibit higher dissolution rate (to non-equivalent level as a pharmaceutical equivalence criteria) of olmesartan medoxomil in an *in vitro* comparative dissolution test, in order to obtain a bioequivalent formulation to the single formulation containing olmesartan medoxomil.

Therefore, it is an object of the present invention to provide a combination formulation having a single dosage form containing olmesartan medoxomil and rosuvastatin as active ingredients, wherein the interaction to *in vivo* absorption is minimized, and wherein the combination formulation is bioequivalent to the single formulation of each of drugs.

### Solution to Problem

In accordance with an aspect of the present invention, there is provided a pharmaceutical composition having a single dosage form comprising a compartment comprising olmesartan medoxomil; and a compartment comprising rosuvastatin or its salt, wherein said compartments are formulated in a separate form.

The pharmaceutical composition of the present invention may have a double-layered tablet form, a tablet form consisting essentially of an inner core and an outer layer, or a pellet-containing capsule form. In an embodiment, the pharmaceutical composition of the present invention has a double-layered tablet form consisting essentially of a layer comprising rosuvastatin or its salt and a layer comprising olmesartan medoxomil. In another embodiment, the pharmaceutical composition of the present invention has a tablet form consisting essentially of an inner core comprising rosuvastatin or its salt and an outer layer comprising Olmesartan medoxomil. In still another embodiment, the pharmaceutical composition of the present invention has a capsule form filled with pellets comprising rosuvastatin or its salt and pellets comprising olmesartan medoxomil.

In the pharmaceutical composition of the present invention, the compartment comprising rosuvastatin or its salt comprises one or more disintegrant selected from the group consisting of crospovidone, low substituted hydroxypropyl cellulose, croscarmellose sodium, and carboxymethylcellulose calcium. The disintegrant may be present in an amount ranging from 2 to 20 % by weight, based on the total weight of the compartment comprising rosuvastatin or its salt.

In the pharmaceutical composition of the present invention, the compartment comprising olmesartan medoxomil comprises one or more disintegrant selected from the group consisting of low substituted hydroxypropyl cellulose, carboxymethylcellulose calcium, croscarmellose sodium, crospovidone, sodium starch glycolate, and pregelatinized starch. In an embodiment, the compartment comprising olmesartan medoxomil comprises 7.5 or more % by weight of low substituted hydroxypropyl cellulose, 5 or more % by weight of carboxymethylcellulose calcium, 15 or more % by weight of croscarmellose sodium, 10 or more % by weight of crospovidone, 5 or more % by weight of sodium starch glycolate, or 5 or more % by weight of pregelatinized starch, based on the total weight of the compartment comprising olmesartan medoxomil. In another embodiment, the compartment comprising olmesartan medoxomil comprises 7.5 to 65 % by weight of low substituted hydroxypropyl cellulose, 5 to 60 % by weight of carboxymethylcellulose calcium, 15 to 30 % by weight of croscarmellose sodium, 10 to 40 % by weight of crospovidone, 5 to 40 % by weight of sodium starch glycolate, or 5 to 60 % by weight of pregelatinized starch, based on the total weight of the compartment comprising olmesartan medoxomil. In still another embodiment, the compartment comprising olmesartan medoxomil comprises 7.5 to 65 % by weight, preferably 10 to 60 % by weight of low substituted hydroxypropyl cellulose, based on the total weight of the compartment comprising olmesartan medoxomii.

### ADVANTAGEOUS EFFECTS

It was found by the present invention that, when rosuvastatin and olmesartan medoxomil are administered in a single matrix formulation, there is drug-drug interaction and/or drug-drug interference between rosuvastatin and olmesartan medoxomil, which results in delaying the *in vivo* release (i.e., dissolution) of rosuvastatin calcium to the gastrointestinal fluid and thus delaying the translocation thereof to the gastrointestinal membrane, thereby inhibiting the absorption of rosuvastatin. In the pharmaceutical composition of the present invention, olmesartan medoxomil and rosuvastatin or its salt are formulated into a combination dosage form having separate compartments, thereby being able to solve the absorption-inhibition problem originated from drug interaction.

And also, the use of a certain disintegrant, i.e., cellulose-type and/or povidone-type disintegrants, provides rapid disintegration and high initial dissolution rate of rosuvastatin or its salt, thereby being able to obtain disintegration and dissolution patterns equivalent to the single formulation containing rosuvastatin or its salt.

And also, it was found by the present invention that, in case of olmesartan medoxomil, a combination formulation comprising rosuvastatin and olmesartan medoxomil should be designed so as to exhibit higher dissolution rate (to non-equivalent level as a pharmaceutical equivalence criteria) of olmesartan medoxomil in an *in vitro* comparative dissolution test, in order to obtain a bioequivalent formulation to the single formulation containing olmesartan medoxomil. That is, the pharmaceutical composition of the present invention exhibits bioequivalence to the single formulation containing olmesartan medoxomil, although it shows non-equivalent *in vitro* dissolution rate.

And also, according to the present invention, olmesartan medoxomil and rosuvastatin or its salt are formulated into a dosage form having separate compartments, which makes it possible to avoid drug interaction in the formulation, thereby having excellent stability.

### Brief Description of Drawings

FIGs 1 and 2 show the blood concentration profiles according to the administration of the double-layered tablet of Example 1 (test formulation) and the co-administration of the reference formulations (FIG 1: Olmetec™ tablet and FIG 2: Crestor™ tablet), respectively.
FIGs 3 and 4 show the blood concentration profiles according to the administration of the single-matrix tablet of Comparative Example 5 (test formulation) and the co-administration of the reference formulation (FIG 3: Olmetec™ tablet and FIG 4: Crestor™ tablet), respectively.
FIGs 5 and 6 show the blood concentration profiles according to the administration of the double-layered tablet of Example 4-1 (test formulation) and the co-administration of the reference formulation (FIG 5: Olmetec™ tablet and FIG 6: Crestor™ tablet), respectively.

### Best Mode for Carrying out the Invention

The present invention provides a pharmaceutical composition in which olmesartan medoxomil and rosuvastatin or its salt are formulated into a combination dosage form having separate compartments. That is, the present invention provides a pharmaceutical composition having a single dosage form comprising a compartment comprising olmesartan medoxomil; and a compartment comprising rosuvastatin or its salt, wherein said compartments are formulated in a separate form.

It was found by the present invention that, when rosuvastatin and olmesartan medoxomil are administered in a single matrix formulation, there is drug-drug interaction and/or drug-drug interference between rosuvastatin and olmesartan medoxomil, which results in delaying the *in vivo* release (i.e., dissolution) of rosuvastatin calcium to the gastrointestinal fluid and thus delaying the translocation thereof to the gastrointestinal membrane, thereby inhibiting the absorption of rosuvastatin. In the pharmaceutical composition of the present invention, olmesartan medoxomil and rosuvastatin or its salt are formulated into a combination dosage form having separate compartments, thereby being able to solve the absorption-inhibition problem originated from drug interaction; and to obtain a combination formulation bioequivalent to the single formulation of each of drugs.

In the pharmaceutical composition of the present invention, the active ingredients, i.e., olmesartan medoxomil and rosuvastatin or its salt, may be used in a therapeutically effect amount. For example, olmesartan medoxomil may be used in an amount of about 5 mg to about 80 mg, preferably about 10 mg to about 40 mg, in a unit formulation (i.e., unit dosage form). And also, rosuvastatin or its salt may be used in an amount of about 2 mg to about 40 mg, preferably about 5 mg to about 20 mg, in a unit formulation (i.e., unit dosage form). The salt of rosuvastatin may be a conventional pharmaceutically acceptable salt, such as calcium salt, hydrochloride, hydrobromide, sulfate, phosphate, acetate, maleate, fumarate, lactate, tartrate, citrate, gluconate, besylate, and camsylate. Preferably, rosuvastatin calcium may be used in the present invention. The pharmaceutical composition of the present invention may be administered once a day, but not limited thereto.

The pharmaceutical composition of the present invention has a combination dosage form having separate compartments, for example, a double-layered tablet form, a tablet form consisting essentially of an inner core and an outer layer, or a pellet-containing capsule form. In an embodiment, the pharmaceutical composition of the present invention has a double-layered tablet form consisting essentially of a layer comprising rosuvastatin or its salt and a layer comprising olmesartan medoxomil. In another embodiment, the pharmaceutical composition of the present invention has a tablet form consisting essentially of an inner core comprising rosuvastatin or its salt and an outer layer (or shell) comprising olmesartan medoxomil. In still another embodiment, the pharmaceutical composition of the present invention has a capsule form filled with pellets comprising rosuvastatin or its salt and pellets comprising olmesartan medoxomil.

Meanwhile, it was found by the present invention that, even when olmesartan medoxomil and rosuvastatin or its salt were formulated into a combination dosage form having separate compartments according to a conventional formulation method, it was difficult to obtain a combination formulation bioequivalent to the single formulation of each of drugs. It is found by the present invention that, when the compartment comprising rosuvastatin or its salt includes a certain disintegrant, i.e., cellulose-type and/or povidone-type disintegrants, in a certain amount, rapid disintegration and high initial dissolution rate of rosuvastatin or its salt can be accomplished, thereby being able to obtain a combination formulation bioequivalent to the single formulation of rosuvastatin or its salt. The disintegrant may be one or more selected from the group consisting of crospovidone (for example, Polyplasdone™, etc.), low substituted hydroxypropyl cellulose, croscarmellose sodium, and carboxymethylcellulose calcium. Preferably, the disintegrant may be a mixture of crospovidone and croscarmellose sodium; or croscarmellose sodium. The disintegrant may be present in an amount ranging from 2 to 20 % by weight, preferably from 3 to 15 % by weight, based on the total weight of the compartment comprising rosuvastatin or its salt. When other disintegrants are used, the dissolution rate of rosuvastatin or its salt is decreased; and/or the amount used is increased, which may cause insufficient compression force during the compressing step, thereby leading to high friability of the resulting formulation (e.g., tablet). In addition, the use of other disintegrants brings about insufficient hardness, which may cause unwanted problems in e.g., packaging, delivery, etc.

And also, it was found by the present invention that, in case of olmesartan medoxomil, a combination formulation comprising rosuvastatin and olmesartan medoxomil should be designed so as to exhibit higher dissolution rate (to non-equivalent level as a pharmaceutical equivalence criteria) of olmesartan medoxomil in an *in vitro* comparative dissolution test, in order to obtain a bioequivalent formulation to the single formulation containing olmesartan medoxomil. That is, the pharmaceutical composition of the present invention exhibits bioequivalence to the single formulation containing olmesartan medoxomil, although it shows non-equivalent *in vitro* dissolution rate. In order to obtain the non-equivalent *in vitro* dissolution rate, the compartment comprising olmesartan medoxomil comprise a certain disintegrant, which may be one or more selected from the group consisting of low substituted hydroxypropyl cellulose, carboxymethylcellulose calcium, croscarmellose sodium, crospovidone, sodium starch glycolate, and pregelatinized starch. In an embodiment, the compartment comprising olmesartan medoxomil comprises 7.5 or more % by weight of low substituted hydroxypropyl cellulose, 5 or more % by weight of carboxymethylcellulose calcium, 15 or more % by weight of croscarmellose sodium, 10 or more % by weight of crospovidone, 5 or more % by weight of sodium starch glycolate, or 5 or more % by weight of pregelatinized starch, based on the total weight of the compartment comprising olmesartan medoxomil. In another embodiment, the compartment comprising olmesartan medoxomil comprises 7.5 to 65 % by weight of low substituted hydroxypropyl cellulose, 5 to 60 % by weight of carboxymethylcellulose calcium, 15 to 30 % by weight of croscarmellose sodium, 10 to 40 % by weight of crospovidone, 5 to 40 % by weight of sodium starch glycolate, or 5 to 60 % by weight of pregelatinized starch, based on the total weight of the compartment comprising olmesartan medoxomil. In still another embodiment, the compartment comprising olmesartan medoxomil comprises 7.5 to 65 % by weight, preferably 10 to 60 % by weight, more preferably about 20 ± 1 % by weight of low substituted hydroxypropyl cellulose, based on the total weight of the compartment comprising olmesartan medoxomil.

The pharmaceutical composition of the present invention, such as a double-layered tablet form, a tablet form consisting essentially of an inner core and an outer layer, or a pellet-containing capsule form, may further comprises one or more excipients conventionally used in the field of pharmaceutics, for example a diluent (or additive), a binder, a lubricant, etc., in addition to said disintegrant. The pharmaceutical composition of the present invention may be also coated with an appropriate coating agent, such as a film-coating agent.

The diluent (or additive) includes lactose (including its hydrate), dextrin, mannitol, sorbitol, starch, microcrystalline cellulose (for example, Celphere™), silicified microcrystalline cellulose (for example, Prosolv™), calcium hydrogen phosphate (including its hydrate), anhydrous calcium hydrogen phosphate, calcium carbonate, saccharides, and a mixture thereof. The binder includes polyvinylpyrrolidone, copovidone, gelatin, starch, sucrose, methyl cellulose, ethyl cellulose, hydroxyethyl cellulose, hydroxypropyl cellulose, hydroxypropyl alkylcellulose (for example, hydroxypropyl methylcellulose), and a mixture thereof. The lubricant includes stearic acid, stearates (for example, magnesium stearate), talc, corn starch, carnauba wax, light anhydrous silicic acid, magnesium silicate, synthetic aluminum silicate, hydrogenated oil, hydrogenated oil, titanium oxide, microcrystalline cellulose, macrogol 4000 or 6000, isopropyl myristate, calcium hydrogen phosphate, and a mixture thereof. The coating agent, for example a film-coating agent, includes a conventional polymer such as Opadry™. The film-coating agent may be used in a minimum amount for providing an appropriate size of the formulation, but not limited thereto.

In an embodiment, the pharmaceutical composition of the present invention having a double-layered tablet form may be prepared by preparing granules containing rosuvastatin and granules containing olmesartan medoxomil, respectively; and then compressing the mixture thereof with a double-layer tablet-press machine. If necessary, the resulting double-layered tablet may be coated with a film-coating agent such as Opadry ™. The granules containing rosuvastatin and the granules containing olmesartan medoxomil may be prepared according to dry granulation methods or wet granulation methods. For example, the granules containing rosuvastatin may be prepared according to a dry granulation method. That is, the granules containing rosuvastatin may be prepared by mixing rosuvastatin calcium, an additive (diluent), a disintegrant, and a lubricant according to a conventional method; and then granulating the mixture with e.g., a roller compactor (TF mini, Vector). And also, the granules containing olmesartan medoxomil may be prepared according to a wet granulation method. That is, the granules containing olmesartan medoxomil may be prepared by mixing olmesartan medoxomil, a binder, an additive (diluent), a disintegrant; granulating the mixture with e.g., a high speed mixer (MIC Developer-5, COMASA); and then drying and sieving the resulting granules.

In another embodiment, the pharmaceutical composition of the present invention having a tablet form consisting essentially of an inner core and an outer layer may by prepared by forming an inner core containing rosuvastatin; optionally forming a film-coating layer; and then compressing the inner core along with granules containing olmesartan medoxomil with e.g., a tablet-press machine (EKO, Korsch). If necessary, the resulting tablet may be coated with a film-coating agent such as Opadry™. The inner core containing rosuvastatin may be prepared by compressing said granules containing rosuvastatin with a rotary tablet-press machine (Piccola D-8, RIVA). If necessary, the resulting inner core may be coated with a film-coating agent such as Opadry™.

In still another embodiment, the pharmaceutical composition of the present invention having a pellet-containing capsule form may prepared by preparing rosuvastatin-containing pellets and olmesartan medoxomil-containing pellets, respectively; and then filling the mixture thereof in a capsule. For example, the rosuvastatin-containing pellets may be prepared by coating beads (e.g., non-pareil beads) with a coating solution in e.g., a fluid bed granulator. The coating solution may be prepared by dissolving rosuvastatin calcium, an additive (diluent), a binder, and a disintegrant in an appropriate solvent, for example a mixed solvent of water and methanol. The coating solution may have a viscosity of 5 mPa·s to 100 mPa·s. Similarly, the olmesartan medoxomil-containing pellets may be prepared by coating beads (e.g., non-pareil beads) with a coating solution in e.g., a fluid bed granulator; and the coating solution may be prepared by dissolving olmesartan medoxomil, an additive (diluent), and a binder in appropriate solvent, for example a mixed solvent of water and methanol.

The present invention will be described in further detail with reference to the following examples and experimental examples. These examples and experimental examples are for illustrative purposes only and are not intended to limit the scope of the present invention.

### Example 1: Preparation of double-layered tablets

Double-layered tablets were prepared according to the components and amounts shown in Table 1. The amounts of the table 1 refer to the amounts per 1 tablet.

### <Step 1> Preparation of granules containing rosuvastatin

Rosuvastatin calcium, lactose monohydrate, Prosolv™, dibasic calcium phosphate dihydrate, crospovidone, croscarmellose sodium, light anhydrous silicic acid, and magnesium stearate (85% of the total amount used in the rosuvastatin-layer) were sieved through a 24 mesh and then mixed. The resulting mixture was granulated using a roller compactor (TF mini, Vector). The obtained granules were sieved through a 24 mesh and then mixed with magnesium stearate pre-sieved though a 35 mesh (15% of the total amount used in the rosuvastatin-layer) to prepare a rosuvastatin-containing granule mixture.

### <Step 2> Preparation of granules containing olmesartan medoxomil

Olmesartan medoxomil, hydroxypropyl cellulose, lactose monohydrate, microcrystalline cellulose, and low substituted hydroxypropyl cellulose were sieved through a 24 mesh and then mixed. The resulting mixture was granulated using a high speed mixer (MIC Developer-5, COMASA). The resulting dry granules were sieved through a 24 mesh and then mixed with magnesium stearate pre-sieved though a 35 mesh and yellow iron oxide pre-sieved through a 80 mesh to prepare a olmesartan medoxomil-containing granule mixture.

### <Step 3> Preparation of double-layered tablets

The rosuvastatin-containing granule mixture prepared in Step 1 and the olmesartan medoxomil-containing granule mixture prepared in Step 2 were compressed with a double-layer tablet-press machine (BB-11, RIVA) to obtain double-layered tablets. The resulting tablets were film-coated with Opadry™ in a pan coating machine (LDCS, VECTOR).

Table 1

**[Table 1]**

| | Components | | Example 1 |
|---|---|---|---|
| Rosuvastatin-co ntaining layer (mg/tablet) | Active ingredient | rosuvastatin calcium | 20.80 |
| | Additive | lactose monohydrate | 119.10 |
| | Additive | Prosolv™ | 42.60 |
| | Additive | dibasic calcium phosphate dihydrate | 21.80 |
| | Disintegrant | crospovidone | 10.70 |
| | Disintegrant | croscarmellose sodium | 6.40 |
| | Lubricant | light anhydrous silicic acid | 4.30 |
| | Lubricant | magnesium stearate | 4.30 |
| Olmesartan medoxomil-cont aining layer (mg/tablet) | Active ingredient | olmesartan medoxomil | 40.00 |
| | Binder | hydroxypropyl cellulose | 10.00 |
| | Additive | lactose monohydrate | 246.27 |
| | Additive | microcrystalline cellulose | 40.00 |
| | Disintegrant | low substituted hydroxypropyl cellulose | 80.00 |
| | Lubricant | magnesium stearate | 3.60 |
| | Lubricant | yellow iron oxide | 0.13 |
| Coating agent | | Opadry™ | 20.00 |

### Example 2: Preparation of tablets containing an inner core and an outer layer

Tablets containing an inner core and an outer layer were prepared according to the components and amounts shown in Table 2. The amounts of the table 2 refer to the amounts per 1 tablet.

### <Step 1> Preparation of inner core-tablets containing rosuvastatin

Rosuvastatin-containing granule mixture was prepared by using the same procedures of Step 1 of Example 1. The inner core-tablets containing rosuvastatin were prepared by compressing the mixture with a rotary tablet-press machine (Piccola D-8, RIVA) to obtain cores and then film-coating the resulting cores with Opadry™ in a pan coating machine (LDCS, VECTOR).

### <Step 2> Preparation of granules containing olmesartan medoxomil

Olmesartan medoxomil-containing granule mixture was prepared by using the same procedures of Step 2 of Example 1.

### <Step 3> Preparation of tablets containing an inner core and an outer layer

The inner core-tablets containing rosuvastatin prepared in Step 1 and the Olmesartan medoxomil-containing granule mixture prepared in Step 2 were compressed with a tablet-press machine (EKO, Korsch) to obtain tablets containing an inner core and an outer layer. The resulting tablets were film-coated with Opadry™ in a pan coating machine (LDCS, VECTOR).

Table 2

**[Table 2]**

| | Components | | Example 2 |
|---|---|---|---|
| Rosuvastatin-co ntaining core (mg/tablet) | Active ingredient | rosuvastatin calcium | 20.80 |
| | Additive | lactose monohydrate | 51.96 |
| | Additive | Prosolv™ | 21.30 |
| | Additive | dibasic calcium phosphate dihydrate | 10.09 |
| | Disintegrant | crospovidone | 5.35 |
| | Disintegrant | croscarmellose sodium | 3.20 |
| | Lubricant | light anhydrous silicic acid | 2.15 |
| | Lubricant | magnesium stearate | 2.15 |
| | Coating agent | Opadry™ | 3.00 |
| Olmesartan medoxomil-cont aining granules (mg/tablet) | Active ingredient | olmesartan medoxomil | 40.00 |
| | Binder | hydroxypropyl cellulose | 10.00 |
| | Additive | lactose monohydrate | 246.27 |
| | Additive | microcrystalline cellulose | 40.00 |
| | Disintegrant | low substituted hydroxypropyl cellulose | 80.00 |
| | Lubricant | magnesium stearate | 3.60 |
| | Coloring agent | yellow iron oxide | 0.13 |
| Coating agent | | Opadry™ | 20.00 |

### Example 2: Preparation of pellet-containing capsules

Pellet-containing capsules were prepared according to the components and amounts shown in Table 3. The amounts of the table 3 refer to the amounts per 1 capsule.

### <Step 1> Preparation of rosuvastatin-containing pellets

A coating solution having a viscosity of about 60 mPa·s was prepared by dissolving rosuvastatin calcium, hydroxypropyl methylcellulose, dibasic calcium phosphate dihydrate, croscarmellose sodium in a mixed solvent of water and methanol (1:2, by weight). Celphere™ (25 to 30 mesh) was coated with the coating solution in a fluid bed granulator to obtain pellets, under the following conditions: 2.7 bar of spray air, 24°C of outlet air temperature, 34°C of inlet air temperature, 20 to 70 m³/hr of flow rate, 28% of outlet air flat.

### <Step 2> Preparation of olmesartan medoxomil-containing pellets

A coating solution having a viscosity of about 45 mPa·s was prepared by dissolving olmesartan medoxomil and hydroxypropyl methylcellulose in a mixed solvent of water and methanol (1:2, by weight). Celphere™ (25 to 30 mesh) was coated with the coating solution in a fluid bed granulator to obtain pellets, under the following conditions: 2.4 bar of spray air, 24°C of outlet air temperature, 34°C of inlet air temperature, 20 to 70 m³/hr of flow rate, 34% of outlet air flat.

### <Step 3> Capsule filling

The rosuvastatin-containing pellets prepared in Step 1 and the olmesartan medoxomil-containing pellets were filled in a # 1 capsule using a capsule-filling machine (DMF1500, DAESAN PHARMATEC) to obtain capsules.

Table 3

**[Table 3]**

| | Components | | Example 3 |
|---|---|---|---|
| Rosuvastatin-containing pellets (mg/capsule) | Active ingredient | rosuvastatin calcium | 20.80 |
| | Additive | Celphere™ | 120 |
| | Binder | hydroxypropyl methylcellulose | 10.50 |
| | Additive | dibasic calcium phosphate dihydrate | 5.20 |
| | Disintegrant | croscarmellose sodium | 3.50 |
| | Solvent | methanol solution | 160.00 |
| Olmesartan medoxomil-containing pellets (mg/capsule) | Active ingredient | olmesartan medoxomil | 40.00 |
| | Additive | Celphere™ | 240.00 |
| | Binder | hydroxypropyl methylcellulose | 20.00 |
| | Solvent | methanol solution | 300.00 |

### Comparative Examples 1 to 4: Preparation of single-matrix tablets

Single-matrix tablets having no disintegrant or having starch, pregelatinized starch, or magnesium aluminum silicate (Veegum™) as a disintegrant were prepared according to the components and amounts shown in Table 4. The amounts of the table 4 refer to the amounts per 1 tablet. The single-matrix tablets were obtained by mixing the rosuvastatin-containing granule mixture and the olmesartan medoxomil-containing granule mixture prepared according to the same procedures of Step 1 and Step 2 of Example 1 respectively for 5 minutes with a polyvinyl bag or a mixer, compressing the resulting mixture with a tablet-press machine (EKO, Korsch) to obtain tablets, and then film-coating the resulting tablets with Opadry™ in a pan coating machine (LDCS, VECTOR).

Table 4

**[Table 4]**

| | Components | | Comparative Example | | | |
|---|---|---|---|---|---|---|
| | | | 1 | 2 | 3 | 4 |
| rosuvastatin-containing granules (mg/tablet) | Active ingredient | rosuvastatin calcium | 20.80 | 20.80 | 20.80 | 20.80 |
| | Additive | lactose monohydrate | 136.20 | 126.20 | 126.20 | 126.20 |
| | Additive | Prosolv™ | 42.60 | 42.60 | 42.60 | 42.60 |
| | Additive | dibasic calcium phosphate dihydrate | 21.80 | 21.80 | 21.80 | 21.80 |
| | Disintegrant | starch | | 10.00 | | |
| | Disintegrant | pregelatinized starch | | | 10.00 | |
| | Disintegrant | Veegum™ | | | | 10.00 |
| | Lubricant | light anhydrous silicic acid | 4.30 | 4.30 | 4.30 | 4.30 |
| | Lubricant | magnesium stearate | 4.30 | 4.30 | 4.30 | 4.30 |
| olmesartan medoxomil-containing granules (mg/tablet) | Active ingredient | olmesartan medoxomil | 40.00 | 40.00 | 40.00 | 40.00 |
| | Binder | hydroxypropyl cellulose | 10.00 | 10.00 | 10.00 | 10.00 |
| | Additive | lactose monohydrate | 326.40 | 246.40 | 246.40 | 246.40 |
| | Additive | microcrystalline cellulose | 40.00 | 40.00 | 40.00 | 40.00 |
| | Disintegrant | starch | | 80.00 | | |
| | Disintegrant | pregelatinized starch | | | 80.00 | |
| | Disintegrant | Veegum™ | | | | 80.00 |
| | Lubricant | magnesium stearate | 3.60 | 3.60 | 3.60 | 3.60 |
| Coating agent | | Opadry™ | 20.00 | 20.00 | 20.00 | 20.00 |

### Comparative Example 5: Preparation of single-matrix tablets

Single-matrix tablets were prepared according to the components and amounts shown in Table 5. The amounts of the table 5 refer to the amounts per 1 tablet. The single-matrix tablets were obtained by mixing the rosuvastatin-containing granule mixture and the olmesartan medoxomil-containing granule mixture prepared according to the same procedures of Step 1 and Step 2 of Example 1 respectively for 5 minutes, compressing the resulting mixture with a tablet-press machine (EKO, Korsch) to obtain tablets, and then film-coating the resulting tablets with Opadry™ in a pan coating machine (LDCS, VECTOR).

Table 5

**[Table 5]**

| | Components | | Comparative Example5 |
|---|---|---|---|
| Rosuvastatin-containing granules (mg/tablet) | Active ingredient | rosuvastatin calcium | 20.80 |
| | Additive | lactose monohydrate | 123.40 |
| | Additive | Prosolv™ | 42.60 |
| | Additive | dibasic calcium phosphate dihydrate | 21.80 |
| | Disintegrant | crospovidone | 10.70 |
| | Lubricant | croscarmellose sodium | 6.40 |
| | Lubricant | light anhydrous silicic acid | 4.30 |
| | Lubricant | magnesium stearate | 4.30 |
| Olmesartan medoxomil-containing granules (mg/tablet) | Active ingredient | olmesartan medoxomil | 40.00 |
| | Binder | hydroxypropyl cellulose | 10.00 |
| | Additive | lactose monohydrate | 246.40 |
| | Additive | microcrystalline cellulose | 40.00 |
| | Disintegrant | low substituted hydroxypropyl cellulose | 80.00 |
| | Lubricant | magnesium stearate | 3.60 |
| Coating agent | | Opadry™ | 20.00 |

### Experimental Example 1: Comparative dissolution test of rosuvastatin

For the formulations prepared in Examples 1 to 3 and Comparative Examples 1to 5, the comparative dissolution tests of rosuvastatin were performed under the following conditions, using a dissolution tester (Vankel VK7025 Vk8000, USA). Crestor™ tablet containing 20 mg of rosuvastatin was used as a reference formulation.

### <Conditions for dissolution test>

Dissolution medium: pH 6.8 buffer (900 mL)
Temperature: 37±0.5°C
Test method: 'Dissolution Test 2 (Paddle Method)' of the Korean Pharmacopeia
(In case of the capsule, a sinker was used)
Paddle rotation rate: 50rpm

### <Conditions for HPLC analysis>

- Detector: UV spectrophotometer (wavelength: 249nm)
- Column: Waters Acquityuplcbeh C18 (2.1 x 50mm, 1.7um)
- Column temperature: 40 °C
- Mobile phase: acetonitrile / buffer (34 / 66, v/v)
- Flow rate: 0.3 mL/min
- Retention time: 2.5 to 3 minutes

The results thereof are presented in the following Table 6.

Table 6

**[Table 6]**

| | | | | | | | | | |
|---|---|---|---|---|---|---|---|---|---|
| Time (min.) | 0 | 5 | 10 | 15 | 30 | 45 | 60 | 90 | 120 |
| Crestor™ tablet 20mg | 0.00 | 69.83 | 91.60 | 94.56 | 96.73 | 98.22 | 99.34 | 100.37 | 100.62 |
| Example 1 | 0.00 | 82.35 | 99.45 | 100.42 | 100.53 | 101.06 | 101.39 | 100.73 | 101.50 |
| Example 2 | 0.00 | 81.35 | 95.45 | 99.42 | 99.53 | 100.06 | 100.39 | 99.73 | 100.50 |
| Example 3 | 0.00 | 75.00 | 90.00 | 96.44 | 96.55 | 97.05 | 97.38 | 96.74 | 97.48 |
| Comparative Example 1 | 0.00 | 50.00 | 77.00 | 88.00 | 98.00 | 98.00 | 98.00 | 98.50 | 99.00 |
| Comparative Example 2 | 0.00 | 50.00 | 75.00 | 86.00 | 96.00 | 96.00 | 96.00 | 96.50 | 97.00 |
| Comparative Example 3 | 0.00 | 45.00 | 76.00 | 87.00 | 98.00 | 99.00 | 99.00 | 99.00 | 99.50 |
| Comparative Example 4 | 0.00 | 40.00 | 80.00 | 90.00 | 99.00 | 100.00 | 100.00 | 100.00 | 100.00 |
| Comparative Example 5 | 0.00 | 86.44 | 99.26 | 99.91 | 99.94 | 99.94 | 100.62 | 100.62 | 100.44 |

As shown in Table 6, in case of the tablet of Comparative Example 1having no disintegrant and the tablets of Comparative Examples 2 to 4 having starch, pregelatinized starch, or magnesium aluminum silicate (Veegum™) as a disintegrant, the dissolution rates of rosuvastatin calcium for the initial 30 minutes were significantly low in comparison with the reference formulation. In contrast, the formulations prepared according to the present invention showed very similar dissolution profiles to the reference formulation. There was observed no significant variation in the dissolution rates of rosuvastatin calcium among the single-matrix tablet (Comparative Example 5) and the formulations of Example 1 to 3.

### Example 4

Double-layered tablets comprising low substituted hydroxypropyl cellulose as a disintegrant in an olmesartan medoxomil-containing layer were prepared by using the same procedures of Example 1 (except that the film-coating process was not performed), according to the components and amounts shown in Table 7. The amounts of the table 7 refer to the amounts per 1 tablet.

Table 7

**[Table 7]**

| | Components | | Example | | | |
|---|---|---|---|---|---|---|
| | | | 4-1 | 4-2 | 4-3 | 4-4 |
| Rosuvastatin-containing layer (mg/tablet) | Active ingredient | rosuvastatin calcium | 20.80 | 20.80 | 20.80 | 20.80 |
| | Additive | lactose monohydrate | 123.40 | 123.40 | 123.40 | 123.40 |
| | Additive | Prosolv™ | 42.60 | 42.60 | 42.60 | 42.60 |
| | Additive | dibasic calcium phosphate dihydrate | 21.80 | 21.80 | 21.80 | 21.80 |
| | Disintegrant | crospovidone | 10.70 | 10.70 | 10.70 | 10.70 |
| | Disintegrant | croscarmellose sodium | 6.40 | 6.40 | 6.40 | 6.40 |
| | Lubricant | light anhydrous silicic acid | 4.30 | 4.30 | 4.30 | 4.30 |
| | Lubricant | magnesium stearate | 4.30 | 4.30 | 4.30 | 4.30 |
| Olmesartan medoxomil-containing layer (mg/tablet) | Active ingredient | olmesartan medoxomil | 40.00 | 40.00 | 40.00 | 40.00 |
| | Binder | hydroxypropyl cellulose | 10.00 | 10.00 | 10.00 | 10.00 |
| | Additive | lactose monohydrate | 305.77 | 294.77 | 74.27 | 53.27 |
| | Additive | microcrystalline cellulose | 40.00 | 40.00 | 40.00 | 40.00 |
| | Disintegrant | low substituted hydroxypropyl cellulose | 21.00(5.0%) | 31.50(7.5%) | 252.00(60.0%) | 273.00(65.0%) |
| | Lubricant | magnesium stearate | 3.60 | 3.60 | 3.60 | 3.60 |

### Example 5

Double-layered tablets comprising carboxymethylcellulose calcium as a disintegrant in an olmesartan medoxomil-containing layer were prepared by using the same procedures of Example 1 (except that the film-coating process was not performed), according to the components and amounts shown in Table 8. The amounts of the table 8 refer to the amounts per 1 tablet.

Table 8

**[Table 8]**

| | Components | | Example | | | |
|---|---|---|---|---|---|---|
| | | | 5-1 | 5-2 | 5-3 | 5-4 |
| Rosuvastatin-containing layer (mg/tablet) | Active ingredient | rosuvastatin calcium | 20.80 | 20.80 | 20.80 | 20.80 |
| | Additive | lactose monohydrate | 123.40 | 123.4 0 | 123.40 | 123.40 |
| | Additive | Prosolv™ | 42.60 | 42.60 | 42.60 | 42.60 |
| | Additive | dibasic calcium phosphate dihydrate | 21.80 | 21.80 | 21.80 | 21.80 |
| | Disintegrant | crospovidone | 10.70 | 10.70 | 10.70 | 10.70 |
| | Disintegrant | croscarmellose sodium | 6.40 | 6.40 | 6.40 | 6.40 |
| | Lubricant | light anhydrous silicic acid | 4.30 | 4.30 | 4.30 | 4.30 |
| | Lubricant | magnesium stearate | 4.30 | 4.30 | 4.30 | 4.30 |
| Olmesartan medoxomil-containing layer (mg/tablet) | Active ingredient | olmesartan medoxomil | 40.00 | 40.00 | 40.00 | 40.00 |
| | Binder | hydroxypropyl cellulose | 10.00 | 10.00 | 10.00 | 10.00 |
| | Additive | lactose monohydrate | 315.77 | 305.77 | 74.27 | 53.27 |
| | Additive | microcrystalline cellulose | 40.00 | 40.00 | 40.00 | 40.00 |
| | Disintegrant | carboxymethylcellulose calcium | 10.50(2.5%) | 21.00(5.0%) | 252.00(60.0%) | 273.00(65.0%) |
| | Lubricant | magnesium stearate | 3.60 | 3.60 | 3.60 | 3.60 |

### Example 6

Double-layered tablets comprising croscarmellose sodium as a disintegrant in an olmesartan medoxomil-containing layer were prepared by using the same procedures of Example 1 (except that the film-coating process was not performed), according to the components and amounts shown in Table 9. The amounts of the table 9 refer to the amounts per 1 tablet.

**<Table 9>**

| | Components | | Example | | | |
|---|---|---|---|---|---|---|
| | | | 6-1 | 6-2 | 6-3 | 6-4 |
| Rosuvastatin-c ontaining layer (mg/tablet) | Active ingredient | rosuvastatin calcium | 20.80 | 20.80 | 20.80 | 20.80 |
| | Additive | lactose monohydrate | 123.40 | 123.40 | 123.40 | 123.40 |
| | Additive | Prosolv™ | 42.60 | 42.60 | 42.60 | 42.60 |
| | Additive | dibasic calcium phosphate dihydrate | 21.80 | 21.80 | 21.80 | 21.80 |
| | Disintegrant | crospovidone | 10.70 | 10.70 | 10.70 | 10.70 |
| | Disintegrant | croscarmellose sodium | 6.40 | 6.40 | 6.40 | 6.40 |
| | Lubricant | light anhydrous silicic acid | 4.30 | 4.30 | 4.30 | 4.30 |
| | Lubricant | magnesium stearate | 4.30 | 4.30 | 4.30 | 4.30 |
| Olmesartan medoxomil-containing layer (mg/tablet) | Active ingredient | olmesartan medoxomil | 40.00 | 40.00 | 40.00 | 40.00 |
| | Binder | hydroxypropyl cellulose | 10.00 | 10.00 | 10.00 | 10.00 |
| | Additive | lactose monohydrate | 284.27 | 263.27 | 200.27 | 179.27 |
| | Additive | microcrystalline cellulose | 40.00 | 40.00 | 40.00 | 40.00 |
| | Disintegrant | croscarmellose sodium | 42.00 (10.0%) | 63.00 (15.0%) | 126.00 (30.0%) | 179.27 (35.0%) |
| | Lubricant | magnesium stearate | 3.60 | 3.60 | 3.60 | 3.60 |

### Example 7

Double-layered tablets comprising crospovidone as a disintegrant in an olmesartan medoxomil-containing layer were prepared by using the same procedures of Example 1 (except that the film-coating process was not performed), according to the components and amounts shown in Table 10. The amounts of the table 10 refer to the amounts per 1 tablet.

Table 10

**[Table 10]**

| | Components | | Example | | | |
|---|---|---|---|---|---|---|
| | | | 7-1 | 7-2 | 7-3 | 7-4 |
| Rosuvastatin-containing layer (mg/tablet) | Active ingredient | rosuvastatin calcium | 20.80 | 20.80 | 20.80 | 20.80 |
| | Additive | lactose monohydrate | 123.40 | 123.40 | 123.40 | 123.40 |
| | Additive | Prosolv™ | 42.60 | 42.60 | 42.60 | 42.60 |
| | Additive | dibasic calcium phosphate dihydrate | 21.80 | 21.80 | 21.80 | 21.80 |
| | Disintegrant | crospovidone | 10.70 | 10.70 | 10.70 | 10.70 |
| | Disintegrant | croscarmellose sodium | 6.40 | 6.40 | 6.40 | 6.40 |
| | Lubricant | light anhydrous silicic acid | 4.30 | 4.30 | 4.30 | 4.30 |
| | Lubricant | magnesium stearate | 4.30 | 4.30 | 4.30 | 4.30 |
| Olmesartan medoxomil-containing layer (mg/tablet) | Active ingredient | olmesartan medoxomil | 40.00 | 40.00 | 40.00 | 40.00 |
| | Binder | hydroxypropyl cellulose | 10.00 | 10.00 | 10.00 | 10.00 |
| | Additive | lactose monohydrate | 294.77 | 284.27 | 158.27 | 137.27 |
| | Additive | microcrystalline cellulose | 40.00 | 40.00 | 40.00 | 40.00 |
| | Disintegrant | crospovidone | 31.50(7.50%) | 42.00(10.0%) | 168.00(40.0%) | 189.00(45.0%) |
| | Lubricant | magnesium stearate | 3.60 | 3.60 | 3.60 | 3.60 |

### Example 8

Double-layered tablets comprising sodium starch glycolate as a disintegrant in an olmesartan medoxomil-containing layer were prepared by using the same procedures of Example 1 (except that the film-coating process was not performed), according to the components and amounts shown in Table 11. The amounts of the table 11 refer to the amounts per 1tablet.

Table 11

**[Table 11]**

| | Components | | Example | | | |
|---|---|---|---|---|---|---|
| | | | 8-1 | 8-2 | 8-3 | 8-4 |
| Rosuvastatin-containing layer (mg/tablet) | Active ingredient | rosuvastatin calcium | 20.80 | 20.80 | 20.80 | 20.80 |
| | Additive | lactose monohydrate | 123.40 | 123.40 | 123.40 | 123.40 |
| | Additive | Prosolv™ | 42.60 | 42.60 | 42.60 | 42.60 |
| | Additive | dibasic calcium phosphate dihydrate | 21.80 | 21.80 | 21.80 | 21.80 |
| | Disintegrant | crospovidone | 10.70 | 10.70 | 10.70 | 10.70 |
| | Disintegrant | croscarmellose sodium | 6.40 | 6.40 | 6.40 | 6.40 |
| | Lubricant | light anhydrous silicic acid | 4.30 | 4.30 | 4.30 | 4.30 |
| | Lubricant | magnesium stearate | 4.30 | 4.30 | 4.30 | 4.30 |
| Olmesartan medoxomil-containing layer (mg/tablet) | Active ingredient | olmesartan medoxomil | 40.00 | 40.00 | 40.00 | 40.00 |
| | Binder | hydroxypropyl cellulose | 10.00 | 10.00 | 10.00 | 10.00 |
| | Additive | lactose monohydrate | 315.77 | 305.77 | 158.27 | 137.27 |
| | Additive | microcrystalline cellulose | 40.00 | 40.00 | 40.00 | 40.00 |
| | Disintegrant | sodium starch glycolate | 10.50(2.5%) | 21.00(5.0%) | 168.00(40.0%) | 189.00(45.0%) |
| | Lubricant | magnesium stearate | 3.60 | 3.60 | 3.60 | 3.60 |

### Example 9

Double-layered tablets comprising pregelatinized starch as a disintegrant in an olmesartan medoxomil-containing layer were prepared by using the same procedures of Example 1(except that the film-coating process was not performed), according to the components and amounts shown in Table 12. The amounts of the table 12 refer to the amounts per 1 tablet.

Table 12

**[Table 12]**

| | Components | | Example | | | |
|---|---|---|---|---|---|---|
| | | | 9-1 | 9-2 | 9-3 | 9-4 |
| Rosuvastatin-containing layer (mg/tablet) | Active ingredient | rosuvastatin calcium | 20.80 | 20.80 | 20.80 | 20.80 |
| | Additive | lactose monohydrate | 123.40 | 123.40 | 123.40 | 123.40 |
| | Additive | Prosolv™ | 42.60 | 42.60 | 42.60 | 42.60 |
| | Additive | dibasic calcium phosphate dihydrate | 21.80 | 21.80 | 21.80 | 21.80 |
| | Disintegrant | crospovidone | 10.70 | 10.70 | 10.70 | 10.70 |
| | Disintegrant | croscarmellose sodium | 6.40 | 6.40 | 6.40 | 6.40 |
| | Lubricant | light anhydrous silicic acid | 4.30 | 4.30 | 4.30 | 4.30 |
| | Lubricant | magnesium stearate | 4.30 | 4.30 | 4.30 | 4.30 |
| Olmesartan medoxomil-containing layer (mg/tablet) | Active ingredient | olmesartan medoxomil | 40.00 | 40.00 | 40.00 | 40.00 |
| | Binder | hydroxypropyl cellulose | 10.00 | 10.00 | 10.00 | 10.00 |
| | Additive | lactose monohydrate | 315.77 | 305.77 | 74.27 | 53.27 |
| | Additive | microcrystalline cellulose | 40.00 | 40.00 | 40.00 | 40.00 |
| | Disintegrant | pregelatinized starch | 10.50(2.5%) | 21.00(5.0%) | 252.00(60.0%) | 273.00(65.0%) |
| | Lubricant | magnesium stearate | 3.60 | 3.60 | 3.60 | 3.60 |

### Experimental Example 2: Comparative dissolution test of olmesartan medoxomil

For the formulations prepared in Examples 1and 4 to 9, the comparative dissolution tests of olmesartan medoxomil were performed by using the same methods of Experimental Example 1, except for using water as a dissolution medium. Olmetec™ tablet containing 40 mg of olmesartan medoxomil was used as a reference formulation. The retention time for olmesartan was 3 to 3.5 minutes. The results thereof are presented in the following Table 13.

Table 13

**[Table 13]**

| | Disintegrant | Amount(w/ w%) | Dissolution rate(5 minutes, %) | Dissolution rate(6 hours, %) |
|---|---|---|---|---|
| | | | | |
| Olmetec™ tablet | | 1 | 21.30 | 51.13 |
| Example 1 | low substituted hydroxypropyl cellulose | 19.05 | 41.73 | 80.61 |
| Example 4-1 | | 5.00 | 16.39 | 61.29 |
| Example 4-2 | | 7.50 | 38.32 | 76.98 |
| Example 4-3 | | 60.00 | 53.39 | 81.68 |
| Example 4-4 | | 65.00 | 57.58 | 82.68 |
| Example 5-1 | carboxymethylcellulose calcium | 2.50 | 23.68 | 81.63 |
| Example 5-2 | | 5.00 | 38.69 | 82.16 |
| Example 5-3 | | 60.00 | 53.26 | 82.65 |
| Example 5-4 | | 65.00 | 56.38 | 83.92 |
| Example 6-1 | croscarmellose sodium | 10.00 | 22.26 | 78.99 |
| Example 6-2 | | 15.00 | 37.69 | 82.95 |
| Example 6-3 | | 30.00 | 51.68 | 83.90 |
| Example 6-4 | | 35.00 | 53.69 | 82.69 |
| Example 7-1 | crospovidone | 7.50 | 21.74 | 78.19 |
| Example 7-2 | | 10.00 | 40.45 | 80.03 |
| Example 7-3 | | 40.00 | 53.96 | 83.28 |
| Example 7-4 | | 45.00 | 58.95 | 84.58 |
| Example 8-1 | sodium starch glycolate | 2.50 | 23.95 | 76.25 |
| Example 8-2 | | 5.00 | 42.26 | 83.29 |
| Example 8-3 | | 40.00 | 54.16 | 82.92 |
| Example 8-4 | | 45.00 | 55.26 | 83.69 |
| Example 9-1 | pregelatinized starch | 2.50 | 18.26 | 73.69 |
| Example 9-2 | | 5.00 | 38.25 | 80.69 |
| Example 9-3 | | 60.00 | 52.68 | 81.69 |
| Example 9-4 | | 65.00 | 57.12 | 84.28 |

In case that the dissolution rate of a reference formulation (i.e., Olmetec™ tablet) in water for the defined time (i.e., 6 hours) is below 85%, the following two requirements should be satisfied in order to meet the pharmaceutical equivalence criteria of the applicable Pharmaceutical Affairs Law: the first requirement that the dissolution rate of the test formulation for 6 hours is located between the dissolution rate of the reference formulation (i.e., Olmetec™ tablet) ± 15% (i.e., 36.13 to 66.13 %); and the second requirement that the dissolution rate thereof for 5 minutes (the nearest time to the time for attaining to about 1/2 (i.e., 25.5%) of the dissolution rate of the reference formulation for 6 hours) is located between the dissolution rate of the reference formulation ± 15% (i.e., 6.30 to 36.30 %).

As shown in Table 13, the dissolution rate of all the formulations (except for the formulation of Example 4-1) for 6 hours exceeded 66.13%. Therefore, all the formulations (except for the formulation of Example 4-1) cannot be regarded as being pharmaceutically equivalent to the reference formulation (i.e., Olmetec™ tablet). And also, in connection with the dissolution rates for 5 minutes, all the formulations except for the formulations of Examples 4-1,5-1,6-1,7-1,8-1, and 9-1 cannot be regarded as being pharmaceutically equivalent to the reference formulation (i.e., Olmetec™ tablet).

### Experimental Example 2: Bioequivalence study

Bioequivalence to the reference formulations (i.e., Olmetec™ tablet and Crestor™ tablet) was evaluated by performing pharmacokinetic studies for the tablet of Example 4-1 (which was evaluated as being pharmaceutically equivalent to the reference formulation from the comparative dissolution test), the tablet of Example 1 (which was evaluated as being pharmaceutically non-equivalent to the reference formulation from the comparative dissolution test), and the tablet of Comparative Example 5. Healthy male volunteers were divided into 2 groups, each group having 12 persons (i.e. n=12). The volunteers of Group 1 were orally administered with the tablet of Example 1; and the volunteers of Group 2 were orally co-administered with Crestor™ tablet containing 20 mg of rosuvastatin and Olmetec™ tablet containing 40 mg of olmesartan medoxomil. And also, in cases of the tablet of Example 4-1 and the tablet of Comparative Example 5, the same administrations and co-administrations were performed respectively, except that each group has 6 persons (i.e., n=6). Blood samples were collected at the time of 0, 0.5, 1, 1.5, 2, 2.5, 3, 4, 5, 6, 8, 10, 12, 16, 24, 48, and 72 after the administration; and then the blood concentrations of olmesartan and rosuvastatin were quantified with ULPC-MS/MS (Waters ACQUITY UPLCTM system), respectively. After the quantifications, bioequivalence between the formulations was evaluated through statistic analyses from the AUC values and the Cmax values of rosuvastatin and olmesartan, which were obtained from the administration of test formulation and the co-administration of the reference formulations, respectively. The bioequivalence evaluation was performed according to the guidance for Bioequivalence Studies of the Food and Drug Administration. Briefly, the AUC values and the Cmax values of rosuvastatin and olmesartan were subject to log-transformation; and then geometric means were calculated. From the geometric means, the respective projected 90% confidence intervals for the geometric mean ratio were calculated. When the projected 90% confidence interval is 0.8 to 1.25, two formulations are regarded as being bioequivalent.

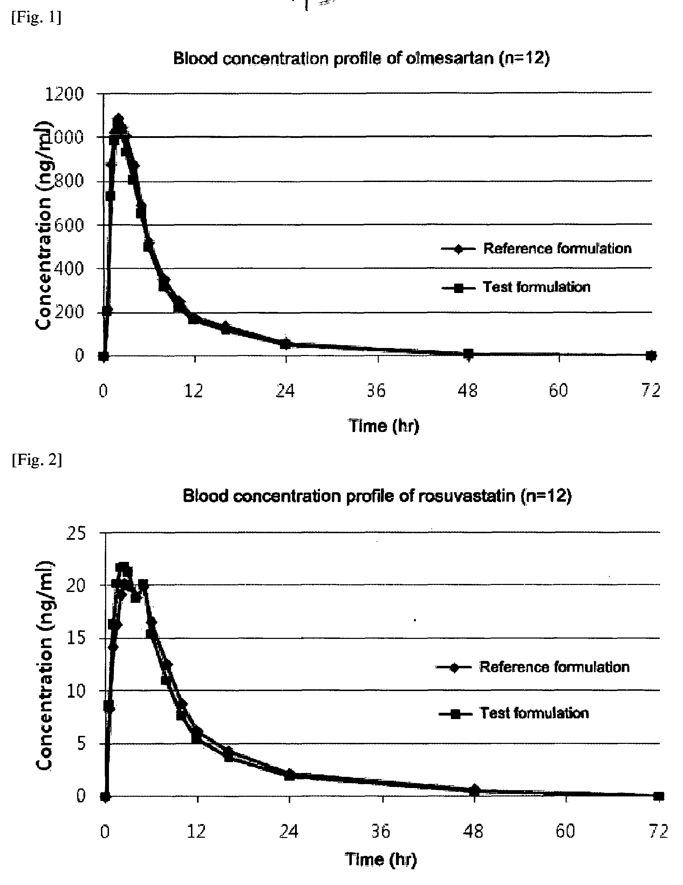

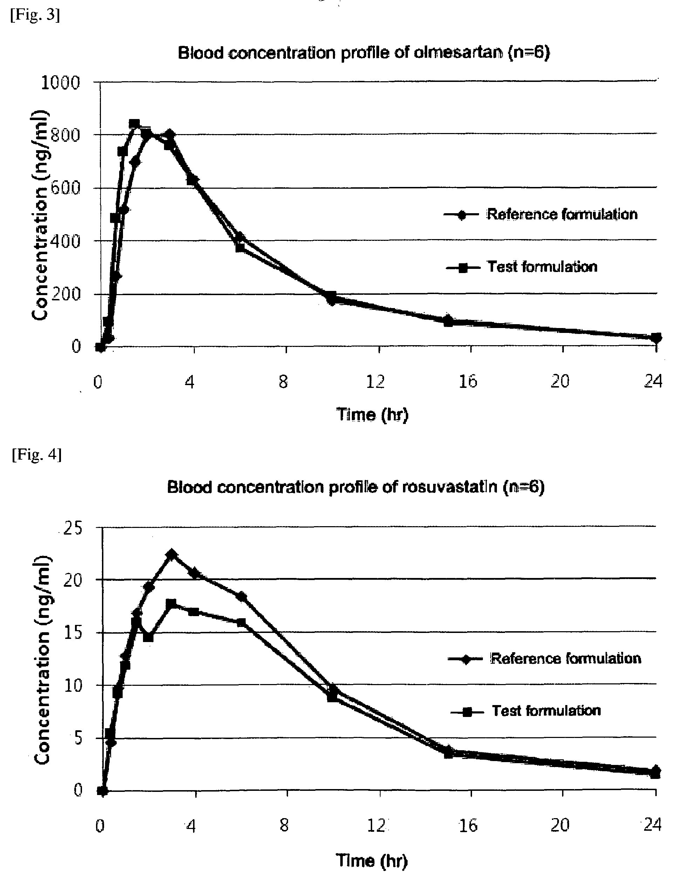

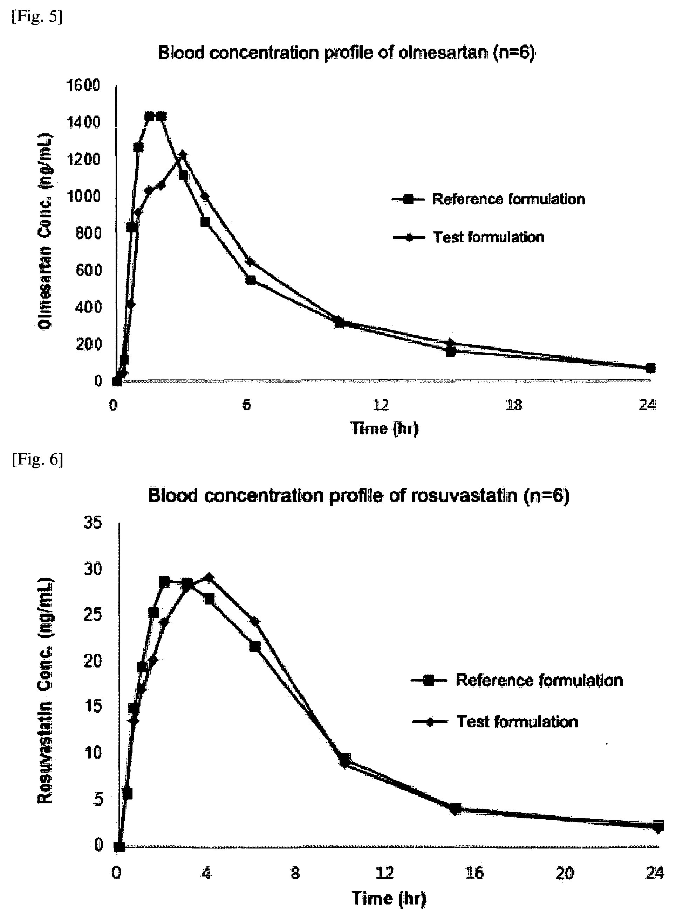

The blood concentration profiles obtained from the pharmacokinetic studies are shown in FIGs 1 to 6. FIGs 1 and 2 show the blood concentration profiles according to the administration of the double-layered tablet of Example 1 (test formulation) and the co-administration of the reference formulations (FIG 1: Olmetec™ tablet and FIG 2: Crestor™ tablet), respectively. FIGs 3 and 4 show the blood concentration profiles according to the administration of the single-matrix tablet of Comparative Example 5 (test formulation) and the co-administration of the reference formulation (FIG 3: Olmetec™ tablet and FIG 4: Crestor™ tablet), respectively. FIGs 5 and 6 show the blood concentration profiles according to the administration of the double-layered tablet of Example 4-1 (test formulation) and the co-administration of the reference formulation (FIG 5: Olmetec™ tablet and FIG 6: Crestor™ tablet), respectively.

The results of the above bioequivalence evaluations are presented in Tables 14 to 16. In the tables 14 to 16, the T/R ratios were calculated by dividing the geometric mean of an evaluation item for the test formulation by the geometric mean of an evaluation item for the reference formulation [i.e., T/R ratio = the geometric mean of an evaluation item for the test formulation / the geometric mean of an evaluation item for the reference formulation]. More than 1 of T/R ratio means that the absorption or the maximum blood concentration of the test formulation is higher than the reference formulation. In contrast, less than 1 of T/R ratio means that the absorption or the maximum blood concentration of the test formulation is lower than the reference formulation. That is, higher deviation between T/R ratio and 1 results in higher probability to regard as being non-bioequivalent.

Table 14

**[Table 14]**

| Projected 90% confidence intervals (the double-layered tablet of Example 1) | | | | |
|---|---|---|---|---|
| PK parameter | Drug | Lower limit | Upper limit | T/R ratio |
| AUC | Olmesartan medoxomil | 0.8716 | 0.9819 | 0.925 |
| | Rosuvastatin calcium | 0.9007 | 1.1169 | 1.002 |
| Cmax | Olmesartan medoxomil | 0.8822 | 1.0284 | 0.952 |
| | Rosuvastatin calcium | 0.9895 | 1.2252 | 1.101 |

Table 15

**[Table 15]**

| Projected 90% confidence intervals (the single-matrix tablet of Comparative Example 5) | | | | |
|---|---|---|---|---|
| PK parameter | Drug | Lower limit | Upper limit | T/R ratio |
| AUC | Olmesartan medoxomil | 0.8687 | 1.0962 | 0.975 |
| | Rosuvastatin calcium | 0.7216 | 1.0045 | 0.851 |
| Cmax | Olmesartan medoxomil | 0.883 | 1.1067 | 0.988 |
| | Rosuvastatin calcium | 0.6382 | 1.1067 | 0.840 |

Table 16

**[Table 16]**

| Projected 90% confidence intervals (the double-layered tablet of Example 4-1) | | | | |
|---|---|---|---|---|
| PK parameter | Drug | Lower limit | Upper limit | T/R ratio |
| AUC | Olmesartan medoxomil | 0.9137 | 1.1743 | 1.035 |
| | Rosuvastatin calcium | 0.8924 | 1.1035 | 0.992 |
| Cmax | Olmesartan medoxomil | 0.7319 | 1.9654 | 0.840 |
| | Rosuvastatin calcium | 0.8912 | 1.1617 | 1.017 |

As shown in Tables 14 and 15, in the tablet of Comparative Example 5, the projected 90% confidence intervals of the AUC and Cmax of rosuvastatin calcium exceeded the range of 0.8 to 1.25, showing very low T/R ratios (i.e., 0.851 and 0.840, respectively). Although there is no significant variation in the dissolution rates of rosuvastatin calcium between the tables of Example 1 and Comparative Example 5 (see Experimental Example 1), the decreases in the AUC and Cmax values is thought to be originated from absorption-delay of rosuvastatin by *in vivo* drug-drug interaction. However, it is expected that an administration of the tablet of Example 1 can provide the same pharmacological effects as a co-administration of the respective reference formulations, without affecting the absorptions of two drugs each other.

And also, as shown in Tables 14 and 16, in the tablet of Example 4-1, the projected 90% confidence intervals of the Cmax of olmesartan medoxomil (i.e., 0.7319 to 0.9654) exceeded the range of 0.8 to 1.25, showing very low T/R ratio (i.e., 0.840). Although the formulation of Example 4-1 was evaluated as being pharmaceutically equivalent to the reference formulation (i.e., Olmetec™ tablet), the formulation of Example 4-1 was not bioequivalent to the reference formulation. However, the formulation of Example 1, which was evaluated as being pharmaceutically non-equivalent to the reference formulation (i.e., Olmetec™ tablet), was bioequivalent to the reference formulation. Therefore, it can be seen that the dissolution rate above the pharmaceutical equivalence criteria (that is, the dissolution rate exceeding the dissolution rate of the reference formulation (Olmetec™ tablet) + 15%) is required in order to meet the bioequivalence criteria. That is, in order to meet the bioequivalence criteria, it is required that the compartment comprising olmesartan medoxomil comprises 7.5 or more % by weight, preferably 10 or more % by weight of low substituted hydroxypropyl cellulose; 5 or more % by weight of carboxymethylcellulose calcium; 15 or more % by weight of croscarmellose sodium; 10 or more % by weight of crospovidone; 5 or more % by weight of sodium starch glycolate; or 5 or more % by weight of pregelatinized starch, based on the total weight of the compartment comprising olmesartan medoxomil. Upper limits of the disintegrants may be controlled to appropriate ranges, considering e.g., hardness of the resulting formulations. For example, the compartment comprising olmesartan medoxomil may comprise 7.5 to 65 % by weight, preferably 10 to 60 % by weight of low substituted hydroxypropyl cellulose; 5 to 60 % by weight of carboxymethylcellulose calcium; 15 to 30 % by weight of croscarmellose sodium; 10 to 40 % by weight of crospovidone; 5 to 40 % by weight of sodium starch glycolate; or 5 to 60 % by weight of pregelatinized starch, based on the total weight of the compartment comprising olmesartan medoxomil.

## Claims

1. A pharmaceutical composition having a single dosage form comprising a compartment comprising olmesartan medoxomil; and a compartment comprising rosuvastatin or its salt,
wherein said compartments are formulated in a separate form,
wherein the compartment comprising olmesartan medoxomil comprises one or more disintegrant selected from the group consisting of low substituted hydroxypropyl cellulose, carboxymethylcellulose calcium, croscarmellose sodium, crospovidone, sodium starch glycolate, and pregelatinized starch, and
wherein the compartment comprising rosuvastatin or its salt comprises one or more disintegrant selected from the group consisting of crospovidone, low substituted hydroxypropyl cellulose, croscarmellose sodium, and carboxymethylcellulose calcium.

2. The pharmaceutical composition according to claim 1, having a double-layered tablet form, a tablet form consisting essentially of an inner core and an outer layer, or a pellet-containing capsule form.

3. The pharmaceutical composition according to claim 1, having a double-layered tablet form consisting essentially of a layer comprising rosuvastatin or its salt and a layer comprising olmesartan medoxomil.

4. The pharmaceutical composition according to claim 1, having a tablet form consisting essentially of an inner core comprising rosuvastatin or its salt and an outer layer comprising olmesartan medoxomil.

5. The pharmaceutical composition according to claim 1, having a capsule form filled with pellets comprising rosuvastatin or its salt and pellets comprising olmesartan medoxomil.

6. The pharmaceutical composition according to claim 1, wherein the disintegrant in the compartment comprising rosuvastatin or its salt is present in an amount ranging from 2 to 20 % by weight, based on the total weight of the compartment comprising rosuvastatin or its salt.

7. The pharmaceutical composition according to claim 1, wherein the compartment comprising olmesartan medoxomil comprises 7.5 or more % by weight of low substituted hydroxypropyl cellulose, 5 or more % by weight of carboxymethylcellulose calcium, 15 or more % by weight of croscarmellose sodium, 10 or more % by weight of crospovidone, 5 or more % by weight of sodium starch glycolate, or 5 or more % by weight of pregelatinized starch, based on the total weight of the compartment comprising olmesartan medoxomil.

8. The pharmaceutical composition according to claim 1, wherein the compartment comprising olmesartan medoxomil comprises 7.5 to 65 % by weight of low substituted hydroxypropyl cellulose, 5 to 60 % by weight of carboxymethylcellulose calcium, 15 to 30 % by weight of croscarmellose sodium, 10 to 40 % by weight of crospovidone, 5 to 40 % by weight of sodium starch glycolate, or 5 to 60 % by weight of pregelatinized starch, based on the total weight of the compartment comprising olmesartan medoxomil.

9. The pharmaceutical composition according to claim 1, wherein the compartment comprising olmesartan medoxomil comprises 7.5 to 65 % by weight of low substituted hydroxypropyl cellulose, based on the total weight of the compartment comprising olmesartan medoxomil.

10. The pharmaceutical composition according to claim 1, wherein the compartment comprising olmesartan medoxomil comprises 10 to 60 % by weight of low substituted hydroxypropyl cellulose, based on the total weight of the compartment comprising olmesartan medoxomil.

## Patentansprüche

1. Pharmazeutische Zusammensetzung mit einer Einzeldosisform, umfassend ein Kompartiment, das Olmesartanmedoxomil umfasst, und ein Kompartiment, das Rosuvastatin oder sein Salz umfasst, wobei die Kompartimente in einer separaten Form formuliert sind,
wobei das Kompartiment, das Olmesartanmedoxomil umfasst, ein oder mehrere Sprengmittel umfasst, die aus der Gruppe bestehend aus gering substituierter Hydroxypropylcellulose, Carboxymethylcellulose-Calcium, Croscarmellose-Natrium, Crospovidon, Natriumstärkeglykolat und vorgelierter Stärke ausgewählt sind, und
wobei das Kompartiment, das Rosuvastatin oder sein Salz umfasst, ein oder mehrere Sprengmittel umfasst, die aus der Gruppe bestehend aus Crospovidon, gering substituierter Hydroxypropylcellulose, Croscarmellose-Natrium und Carboxymethylcellulose-Calcium ausgewählt sind.

2. Pharmazeutische Zusammensetzung nach Anspruch 1 mit einer Doppelschicht-Tablettenform, einer Tablettenform, die im Wesentlichen aus einem inneren Kern und einer äußeren Schicht besteht, oder einer pellethaltigen Kapselform.

3. Pharmazeutische Zusammensetzung nach Anspruch 1 mit einer Doppelschicht-Tablettenform, die im Wesentlichen aus einer Schicht, die Rosuvastatin oder sein Salz umfasst, und einer Schicht, die Olmesartanmedoxomil umfasst, besteht.

4. Pharmazeutische Zusammensetzung nach Anspruch 1 mit einer Tablettenform, die im Wesentlichen aus einem inneren Kern, der Rosuvastatin oder sein Salz umfasst, und einer äußeren Schicht, die Olmesartanmedoxomil umfasst, besteht.

5. Pharmazeutische Zusammensetzung nach Anspruch 1 mit einer Kapselform, die mit Pellets, die Rosuvastatin oder sein Salz umfassen, und Pellets, die Olmesartanmedoxomil umfassen, gefüllt ist.

6. Pharmazeutische Zusammensetzung nach Anspruch 1, wobei das Sprengmittel in dem Kompartiment, das Rosuvastatin oder sein Salz umfasst, in einer Menge vorliegt, die von 2 bis 20 Gew.-% reicht, bezogen auf das Gesamtgewicht des Kompartiments, das Rosuvastatin oder sein Salz umfasst.

7. Pharmazeutische Zusammensetzung nach Anspruch 1, wobei das Kompartiment, das Olmesartanmedoxomil umfasst, 7,5 Gew.-% oder mehr gering substituierter Hydroxypropylcellulose, 5 Gew.-% oder mehr Carboxymethylcellulose-Calcium, 15 Gew.-% oder mehr Croscarmellose-Natrium, 10 Gew.-% oder mehr Crospovidon, 5 Gew.-% oder mehr Natriumstärkeglykolat oder 5 Gew.-% oder mehr vorgelierter Stärke umfasst, bezogen auf das Gesamtgewicht des Kompartiments, das Olmesartanmedoxomil umfasst.

8. Pharmazeutische Zusammensetzung nach Anspruch 1, wobei das Kompartiment, das Olmesartanmedoxomil umfasst, 7,5 bis 65 Gew.-% gering substituierter Hydroxypropylcellulose, 5 bis 60 Gew.-% Carboxymethylcellulose-Calcium, 15 bis 30 Gew.-% Croscarmellose-Natrium, 10 bis 40 Gew.-% Crospovidon, 5 bis 40 Gew.-% Natriumstärkeglykolat oder 5 bis 60 Gew.-% vorgelierter Stärke umfasst, bezogen auf das Gesamtgewicht des Kompartiments, das Olmesartanmedoxomil umfasst.

9. Pharmazeutische Zusammensetzung nach Anspruch 1, wobei das Kompartiment, das Olmesartanmedoxomil umfasst, 7,5 bis 65 Gew.-% gering substituierter Hydroxypropylcellulose umfasst, bezogen auf das Gesamtgewicht des Kompartiments, das Olmesartanmedoxomil umfasst.

10. Pharmazeutische Zusammensetzung nach Anspruch 1, wobei das Kompartiment, das Olmesartanmedoxomil umfasst, 10 bis 60 Gew.-% gering substituierter Hydroxypropylcellulose umfasst, bezogen auf das Gesamtgewicht des Kompartiments, das Olmesartanmedoxomil umfasst.

## Revendications

1. Composition pharmaceutique ayant une forme galénique unitaire comprenant un compartiment comprenant de l'olmésartan médoxomil; et un compartiment comprenant de la rosuvastatine ou son sel, dans laquelle lesdits compartiments sont formulés sous forme séparée,
dans laquelle le compartiment comprenant de l'olmésartan médoxomil comprend un ou plusieurs agents de délitement choisis dans le groupe formé par l'hydroxypropylcellulose faiblement substituée, la carboxyméthylcellulose calcique, la croscarmellose sodique, la crospovidone, le glycolate d'amidon sodique et l'amidon prégélatinisé, et
dans laquelle le compartiment comprenant la rosuvastatine ou son sel comprend un ou plusieurs agents de délitement choisis dans le groupe formé par la crospovidone, l'hydroxypropylcellulose faiblement substituée, la croscarmellose sodique et la carboxyméthylcellulose calcique.

2. Composition pharmaceutique selon la revendication 1, ayant une forme de comprimé bicouche, une forme de comprimé étant essentiellement constituée d'un noyau intérieur et d'une couche extérieure, ou une forme de capsule contenant des pellets.

3. Composition pharmaceutique selon la revendication 1, ayant une forme de comprimé bicouche essentiellement constituée d'une couche comprenant de la rosuvastatine ou son sel et d'une couche comprenant de l'olmésartan médoxomil.

4. Composition pharmaceutique selon la revendication 1, ayant une forme de comprimé essentiellement constituée d'un noyau intérieur comprenant de la rosuvastatine ou son sel et d'une couche extérieure comprenant de l'olmésartan médoxomil.

5. Composition pharmaceutique selon la revendication 1, ayant une forme de capsule remplie de pellets comprenant de la rosuvastatine ou son sel et de pellets comprenant de l'olmésartan médoxomil.

6. Composition pharmaceutique selon la revendication 1,
dans laquelle l'agent de délitement dans le compartiment comprenant de la rosuvastatine ou son sel est présent dans une quantité allant de 2 à 20 % en poids, par rapport au poids total du compartiment comprenant de la rosuvastatine ou son sel.

7. Composition pharmaceutique selon la revendication 1,
dans laquelle le compartiment comprenant de l'olmésartan médoxomil comprend 7,5 % en poids ou plus d'hydroxypropyl-cellulose faiblement substituée, 5 % en poids ou plus de carboxyméthylcellulose calcique, 15 % en poids ou plus de croscarmellose sodique, 10 % en poids ou plus de crospovidone, 5 % en poids ou plus de glycolate d'amidon sodique ou 5 % en poids ou plus d'amidon prégélatinisé, par rapport au poids total du compartiment comprenant de l'olmésartan médoxomil.

8. Composition pharmaceutique selon la revendication 1,
dans laquelle le compartiment comprenant de l'olmésartan médoxomil comprend 7,5 à 65 % en poids d'hydroxypropyl-cellulose faiblement substituée, 5 à 60 % en poids de carboxyméthylcellulose calcique, 15 à 30 % en poids de croscarmellose sodique, 10 à 40 % en poids de crospovidone, 5 à 40 % en poids de glycolate d'amidon sodique ou 5 à 60 % en poids d'amidon prégélatinisé, par rapport au poids total du compartiment comprenant de l'olmésartan médoxomil.

9. Composition pharmaceutique selon la revendication 1,
dans laquelle le compartiment comprenant de l'olmésartan médoxomil comprend 7,5 à 65 % en poids d'hydroxypropyl-cellulose faiblement substituée, par rapport au poids total du compartiment comprenant de l'olmésartan médoxomil.

10. Composition pharmaceutique selon la revendication 1,
dans laquelle le compartiment comprenant de l'olmésartan médoxomil comprend 10 à 60 % en poids d'hydroxypropyl-cellulose faiblement substituée, par rapport au poids total du compartiment comprenant de l'olmésartan médoxomil.
